(19) Europäisches Patentamt
European Patent Office
Office européen des brevets

(11) Veröffentlichungsnummer: **0 025 949**
**B1**

# (12) EUROPÄISCHE PATENTSCHRIFT

(45) Veröffentlichungstag der Patentschrift: **20.07.83**

(21) Anmeldenummer: **80105459.4**

(22) Anmeldetag: **12.09.80**

(51) Int. Cl.³: **C 07 D 249/08**, A 01 N 43/64, A 01 N 47/10, A 01 N 47/20 // C07C49/255, C07C49/16, C07C49/167, C07C143/68

## (54) Acylierte Triazolyl-gamma-fluorpinakolyl-Derivate, Verfahren zu ihrer Herstellung und ihre Verwendung als Fungizide.

(30) Priorität: **24.09.79 DE 2938534**

(43) Veröffentlichungstag der Anmeldung: **01.04.81 Patentblatt 81/13**

(45) Bekanntmachung des Hinweises auf die Patenterteilung: **20.07.83 Patentblatt 83/29**

(84) Benannte Vertragsstaaten: **AT BE CH DE FR GB IT LI NL SE**

(56) Entgegenhaltungen:
EP-A-0 004 303
EP-A-0 006 538
EP-A-0 019 130
DE-A-2 811 916

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal 1 (DE)**
Erfinder: **Büchel, Karl Heinz, Prof. Dr., Haus an der Dachsdelle Dabringhausener Strasse 42, D-5093 Burscheid (DE)**
Erfinder: **Stetter, Jörg, Dr., Gellertweg 4, D-5600 Wuppertal 1 (DE)**
Erfinder: **Frohberger, Paul-Ernst, Dr., Willi Baumeister Strasse 5, D-5090 Leverkusen 1 (DE)**
Erfinder: **Brandes, Wilhelm, Dr., Eichendorffstrasse 3, D-5623 Leichlingen (DE)**

EP 0 025 949 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Acylierte Triazolyl-γ-fluorpinakolylderivate, Verfahren zu ihrer Herstellung und ihre Verwendung als Fungizide

Die Erfindung betrifft neue acylierte Triazolyl-γ-fluorpinakolylderivate, mehrere Verfahren zu ihrer Herstellung und ihre Verwendung als Fungizide.

Es ist bereits bekannt geworden, dass acylierte 1-Triazolyl-2-hydroxybutanderivate, wie insbesondere im Phenyl teil substituierte 2-Acyloxy-3,3-dimethyl-1-phenoxy-1-(1,2,4-triazol-1-yl)-butane, gute fungizide Eigenschaften aufweisen (vgl. DE-OS Nr. 2600799). Deren Wirkung ist jedoch, insbesondere bei niedrigen Aufwandmengen und -konzentrationen, nicht immer ganz befriedigend.

Es wurden neue acylierte Triazolyl-γ-fluorpinakolylderivate der allgemeinen Formel I

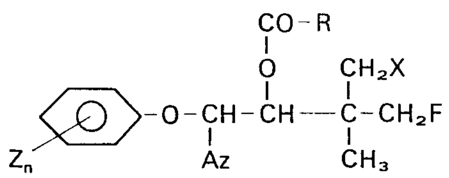

(I)

in welcher

Az für 1,2,4-Triazolyl-1-yl oder 1,2,4-Triazolyl-4-yl steht,

R für Alkyl mit 1 bis 6, Alkenyl und Alkinyl mit jeweils 2 bis 4 Kohlenstoffatomen, Halogenalkyl mit 1 bis 3 Kohlenstoff- und 1 bis 5 Halogenatomen, Alkoxy und Alkoxyalkyl mit 1 bis 4 Kohlenstoffatomen in den Alkylteilen steht, ferner für Cyclohexyl steht, sodann für Phenyl, Phenylalkyl und Phenoxyalkyl mit jeweils bis zu 2 Kohlenstoffatomen im Alkylteil steht, wobei die drei letztgenannten Gruppen im Phenylteil substituiert sein können durch Halogen, Alkyl und Alkoxy mit jeweils 1 bis 2 Kohlenstoffatomen, weiterhin für Alkylamino mit 1 bis 12 Kohlenstoffatomen und für Dimethyl- oder Diäthylamino, Halogenalkylamino mit bis zu 4 Kohlenstoff- und 5 Halogenatomen steht, für Alkoxycarbonylamino und Alkoxyalkylamino mit jeweils 1 bis 4 Kohlenstoffatomen in jedem Alkylteil und endlich für Phenylamino steht, welches gegebenenfalls durch Halogen, Alkyl mit bis zu 4 Alkoxy, und Alkylthio mit bis zu 2 Kohlenstoffatomen und durch Halogenalkyl mit bis zu 2 Kohlenstoff- und 5 Halogenatomen substituiert sein kann;

X für Wasserstoff oder Fluor steht,

Z für Halogen, Cyano, Nitro und Alkyl mit bis zu 4 Kohlenstoffatomen, für Cyclohexyl, für Halogenalkyl mit bis zu 2 Kohlenstoff- und 5 Halogenatomen, für Alkoxy und Alkylthio mit bis zu 2 und Alkoxycarbonyl mit insgesamt bis zu 5 Kohlenstoffatomen steht und weiterhin für Phenyl, Benzyl und Phenoxy steht, wobei die drei letztgenannten Reste gegebenenfalls durch Halogen substituiert sein können, und

n für ganze Zahlen von 0 bis 3 steht,

und deren physiologisch verträglichen Säureadditionssalze und Metallsalzkomplexe gefunden.

Die Verbindungen der allgemeinen Formel I besitzen zwei asymmetrische Kohlenstoffatome; sie können deshalb in der erythro- wie in der threo-Form vorliegen. In beiden Fällen liegen sie vorwiegend als Racemate vor.

Weiterhin wurde gefunden, dass man die acylierten Triazolyl-γ-fluorpinakolylderivate der allgemeinen Formel I erhält, wenn man 1-Triazolyl-Formel I erhält, wenn man 1-Triazolyl-2-hydroxybutanderivate der allgemeinen Formel II

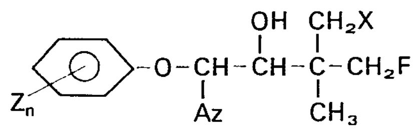

(II)

in welcher

Az, X, Z und n die oben angegebene Bedeutung haben,

a) mit Säurehalogeniden der allgemeinen Formel III

$$Hal-CO-R \qquad (III)$$

in welcher

R die oben angegebene Bedeutung hat, und

Hal für Halogen, insbesondere Chlor oder Brom steht,

gegebenenfalls in Gegenwart eines Lösungsmittels und gegebenenfalls in Gegenwart eines Säurebindemittels umsetzt, oder

b) mit Säureanhydriden der allgemeinen Formel IV

$$R-CO-O-CO-R \qquad (IV)$$

in welcher

R die oben angegebene Bedeutung hat,

in Gegenwart eines Lösungsmittels und gegebenenfalls in Gegenwart eines Katalystors umsetzt, oder

c) mit Ketenen der allgemeinen Formel V

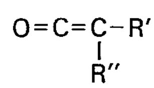

(V)

in welcher

R' für Wasserstoff, für Alkyl mit bis zu 5 oder für Alkenyl oder Alkoxy mit bis zu 3 Kohlenstoffatomen steht, ferner für Phenyl steht, welches durch Halogen, Alkyl und Alkoxy mit bis zu 2 Kohlenstofftomen substituiert sein kann, sodann für Halogenmethyl mit 1 bis 3 Fluor- und/oder Chloratomen steht, und schliesslich für Chlor oder Brom steht, und

R'' die unter R' genannten Bedeutungen annehmen kann, jedoch mit der Massgabe, dass die Summe der Kohlenstoffatome in den Alkyl-, Alkenyl- und Alkoxyresten für R' und R'' gemeinsam um die Zahl 1 unter der Zahl derjenigen Kohlenstoffatome bleiben muss, die oben bei der R-Definition für die genannten Reste angegeben ist, und dass höchstens einer der Reste R' und R'' für gegebenenfalls wie angegeben substituiertes Phenyl oder Halogenmethyl stehen darf, in Gegenwart eines Lösungsmittels und gegebenenfalls in Gegenwart eines Katalysators umsetzt, oder

d) mit Isocyanaten der allgemeinen Formel VI

$$O{=}C{=}N{-}R''' \qquad (VI)$$

in welcher

R''' für Alkyl mit bis zu 12, für Halogenalkyl mit bis zu 4 Kohlenstoff- und bis zu 5 Halogenatomen steht, für Alkoxycarbonyl oder Alkoxyalkyl mit jeweils bis zu 4 Kohlenstoffatomen in jedem Alkylteil steht, oder für Phenyl steht, welches durch Halogen, durch Alkyl mit bis 4, Alkoxy und Alkylthio mit bis zu 2 Kohlenstoffatomen und durch Halogenalkyl mit bis zu 2 Kohlenstoff- und bis zu 5 Halogenatomen substituiert sein kann,

in Gegenwart eines Lösungsmittels und gegebenenfalls in Gegenwart eines Katalysators umsetzt.

Weiterhin können die erfindungsgemässen acylierten Triazolyl-γ-fluorpinalkolylderivate der allgemeinen Formel I durch Umsetzen mit Säuren in die Salze überführt werden, bzw. können durch Reaktion mit Metallsalzen die entsprechenden Metallsalzkomplexe erhalten werden.

Die neuen acylierten Triazolyl-γ-fluorpinakolylderivate weisen starke fungizide Eigenschaften auf. Dabei zeigen überraschenderweise die erfindungsgemässen Verbindungen eine erheblich höhere Wirkung, als die aus dem Stand der Technik bekannten acylierten Triazolyl-2-hydroxybutanderivate, die chemisch und wirkungsmässig naheliegende Verbindungen sind. Die erfindungsgemässen Stoffe stellen somit eine Bereicherung der Technik dar.

Die erfindungsgemässen acylierten Triazolyl-γ-fluorpinakolylderivate sind durch die allgemeine Formel I allgemein definiert. Bevorzugt sind diejenigen Verbindungen, in denen R für Methyl, Äthyl, Isobutyl, Chlormethyl, Dichlormethyl, Chloräthyl, Chlorpropyl, Methacryl, Cyclohexyl, gegebenenfalls einfach oder mehrfach substituiertes Phenyl, Benzyl oder Phenoxymethyl mit Chlor, Brom, Methyl oder Benzyl oder Phenoxymethyl mit Chlor, Brom, Methyl oder Methoxy als Substituenten, ferner für Methoxy, Äthoxy, Isopropoxy, Butoxy oder Isobutoxy, Methyl- und Äthylamino, Dimethylamino, Phenylamino, Chlorphenylamino, Chloräthylamino, Methoxycarbonylamino, Äthoxycarbonylamino und Methoxymethylamino steht, X für Wasserstoff oder Fluor steht; Z für Chlor, Brom, Methyl, Äthyl, Cyclohexyl, Methoxy, Methylthio, Trifluormethyl, Methoxycarbonyl, Cyano, Nitro sowie gegebenenfalls durch Chlor substituiertes Phenyl, Benzyl oder Phenoxy steht und n für 0, 1 oder 2 steht.

Im einzelnen seien ausser den Herstellungsbeispielen und den Beispielen der Tabelle 1 folgende Verbindungen der allgemeinen Formel I genannt (Az steht dabei sowohl für 1,2,4-Triazol-1-yl als auch für 1,2,4-Triazol-4-yl):

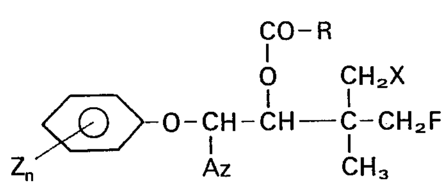

(I)

| R | X | $Z_n$ |
|---|---|---|
| $-CHCl_2$ | H | 4-Cl |
| $-CH_2Cl$ | H | 4-Cl |
| $-CH_3$ | H | 4- 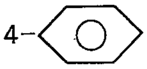 |
| -NH- 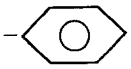 | H | 4- 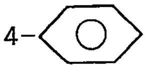 |
| -NH- 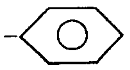 -Cl | H | 4- 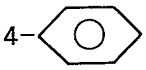 |
| $-NHCH_3$ | H | 4- 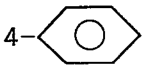 |
| $-NHCH_3$ | H | 4-Cl; $2-CH_3$ |
| $-NHCH_3$ | H | 2-Cl |
| $-NHC_2H_5$ | H | 4- 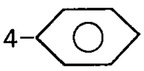 |
| $-C_4H_9-i$ | H | 4-Br |
| $-CH_3$ | H | $2,4-Cl_2$ |
| $-C_2H_5$ | H | $2,4-Cl_2$ |
| $-CH_3$ | H | $4-OCH_3$ |
| $-CH_2Cl$ | H | $3-CF_3$ |
| $-CHCl_2$ | H | $4-CO-OCH_3$ |
| $-NHCH_2OCH_3$ | H | 4- 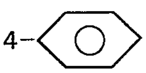 |
| $-NHCH_2OC_2H_5$ | H | 4- 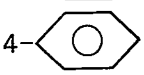 |
| $-NH-COOCH_3$ | H | 4- 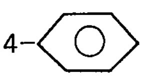 |
| $-NH-COOC_2H_5$ | H | 4- 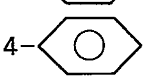 |
| $-N(CH_3)_2$ | H | 4- 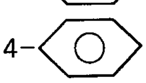 |
| $-OCH_3$ | H | 4- 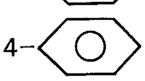 |
| $-CH_3$ | H | 4- 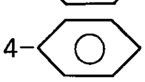 -Cl |
| $-CH_3$ | H | 4-O- 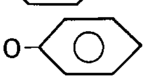 |
| $-CH_3$ | H | 4-O- 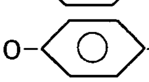 -Cl |
| $-CH_3$ | H | 4-CN |
| $-CH_3$ | H | $4-NO_2$ |
| $-CH_2OC_2H_5$ | H | $2,4-Cl_2$ |
| $-NHCH(CH_3)_2$ | H | $2,4-Cl_2$ |
| $-NH-CH_2OCH_3$ | H | $2,4-Cl_2$ |
| $-OC_2H_5$ | H | $2,4-Cl_2$ |
| $-C(CH_3)=CH_2$ | H | $2,4-Cl_2$ |

| R | X | $Z_n$ |
|---|---|---|
| $-CH_2-CH(CH_3)_2$ | H | 2,4-$Cl_2$ |
| 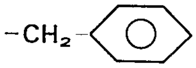 | H | 2,4-$Cl_2$ |
| $-CH_2-CH_2Cl$ | H | 2,4-$Cl_2$ |
| $-CH_2-CH_2-CH_2Cl$ | H | 2,4-$Cl_2$ |
| 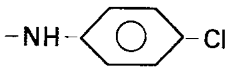 | H | 2,4-$Cl_2$ |
| $-CHCl_2$ | H | 2,4-$Cl_2$ |
| $-CH_2Cl$ | H | 2,4-$Cl_2$ |
| 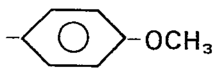 | H | 2,4-$Cl_2$ |
| 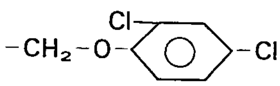 | H | 2,4-$Cl_2$ |
| 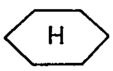 | H | 2,4-$Cl_2$ |
| 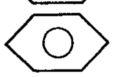 | H | 2,4-$Cl_2$ |
| 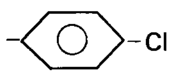 | H | 2,4-$Cl_2$ |
| $-CH_2OC_2H_5$ | H | 4-Cl |
| $-NH-CH(CH_3)_2$ | H | 4-Cl |
| $-NH-CH_2OCH_3$ | H | 4-Cl |
| $-OC_2H_5$ | H | 4-Cl |
| $-C(CH_3)=CH_2$ | H | 4-Cl |
| $-CH_2CH(CH_3)_2$ | H | 4-Cl |
| 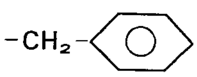 | H | 4-Cl |
| $-CH_2-CH_2Cl$ | H | 4-Cl |
| $-CH_2-CH_2-CH_2Cl$ | H | 4-Cl |
| 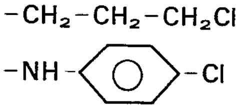 | H | 4-Cl |
| 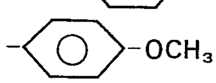 | H | 4-Cl |
| 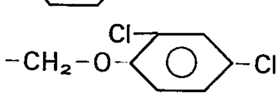 | H | 4-Cl |
| 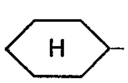 | H | 4-Cl |
| 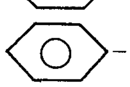 | H | 4-Cl |
| 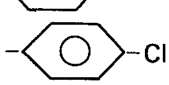 | H | 4-Cl |
| $-NHCH_3$ | F | 2,4-$Cl_2$ |
| $-NHCH_3$ | F | 4-Cl |
| $-CH_3$ | F | 4- 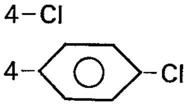 |

| R | X | $Z_n$ |
|---|---|---|
| $-NHCH_3$ | F | 4- 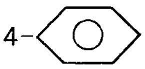 |
| $-NHC_2H_5$ | F | 4- 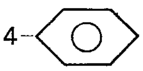 |
| $-NHCH_2OCH_3$ | F | 4- 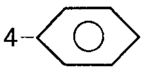 |
| $-NH-CH(CH_3)_2$ | F | 4- 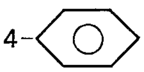 |
| $-CH_2OC_2H_5$ | F | 4-Cl |
| $-NH-CH(CH_3)_2$ | F | 4-Cl |
| $-NHCH_2OCH_3$ | F | 4-Cl |
| $-OC_2H_5$ | F | 4-Cl |
| $-C(CH_3)=CH_2$ | F | 4-Cl |
| $-CH_2-CH(CH_3)_2$ | F | 4-Cl |
| 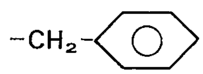 | F | 4-Cl |
| $-CH_2-CH_2Cl$ | F | 4-Cl |
| $-CH_2-CH_2-CH_2Cl$ | F | 4-Cl |
| 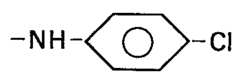 | F | 4-Cl |
| $-CHCl_2$ | F | 4-Cl |
| $-CH_2Cl$ | F | 4-Cl |
| 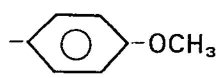 | F | 4-Cl |
| 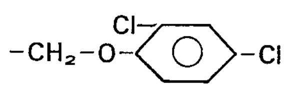 | F | 4-Cl |
| 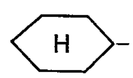 | F | 4-Cl |
| 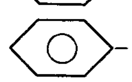 | F | 4-Cl |
| 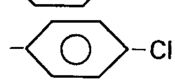 | F | 4-Cl |
| $-CH_2OC_2H_5$ | F | 2,4-$Cl_2$ |
| $-NH-CH(CH_3)_2$ | F | 2,4-$Cl_2$ |
| $-NH-CH_2OCH_3$ | F | 2,4-$Cl_2$ |
| $-OC_2H_5$ | F | 2,4-$Cl_2$ |
| $-C(CH_3)=CH_2$ | F | 2,4-$Cl_2$ |
| $-CH_2-CH(CH_3)_2$ | F | 2,4-$Cl_2$ |
| 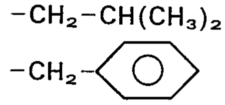 | F | 2,4-$Cl_2$ |
| $-CH_2-CH_2Cl$ | F | 2,4-$Cl_2$ |
| $-CH_2-CH_2-CH_2Cl$ | F | 2,4-$Cl_2$ |
| 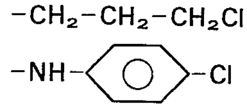 | F | 2,4-$Cl_2$ |
| $-CHCl_2$ | F | 2,4-$Cl_2$ |

| R | X | $Z_n$ |
|---|---|---|
| $-CH_2Cl$ | F | 2,4-$Cl_2$ |
| 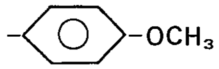 | F | 2,4-$Cl_2$ |
| 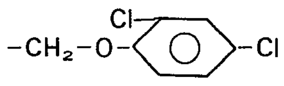 | F | 2,4-$Cl_2$ |
| 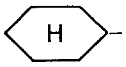 | F | 2,4-$Cl_2$ |
| 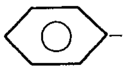 | F | 2,4-$Cl_2$ |
| 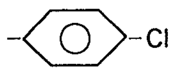 | F | 2,4-$Cl_2$ |

Verwendet man beispielsweise 1-(4-Chlorphenoxy)-1-(1,2,4-triazol-1-yl)-3,3-dimethyl-4-fluorbutan-2-ol und Dichloracetylchlorid als Ausgangsstoffe, so kann der Reaktionsablauf durch das folgende Formelschema wiedergegeben werden (Verfahren a) -

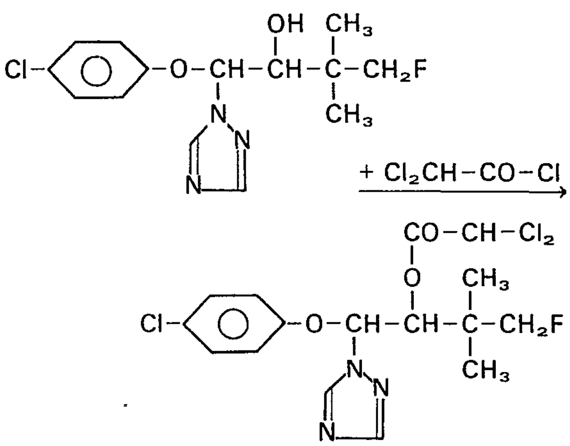


Verwendet man beispielsweise 1-(4-Chlorphenoxy)-1-(1,2,4-triazol-1-yl)-3,3-dimethyl-4-fluorbutan-2-ol und Acetanhydrid als Ausgangsstoffe, so kann der Reaktionsablauf durch das folgende Formelschema wiedergegeben werden (Verfahren b):

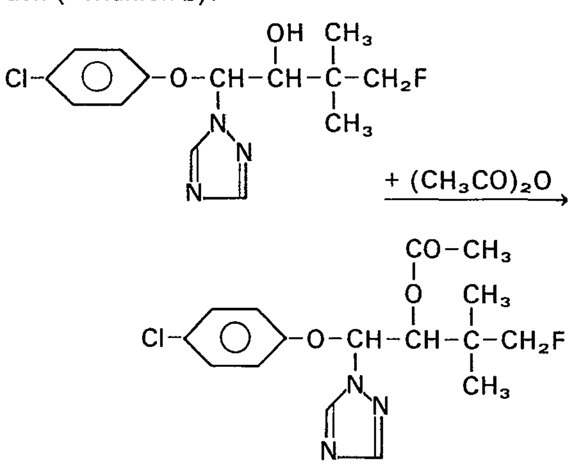


Verwendet man beispielsweise 1-(2,4-Dichlorphenoxy)-1-(1,2,4-triazol-1-yl)-3,3-dimethyl-4-fluorbutan-2-ol und 4-Chlorphenylisocyanat als Ausgangsstoffe, so kann der Reaktionsablauf durch das folgende Formelschema wiedergegeben werden (Verfahren d):

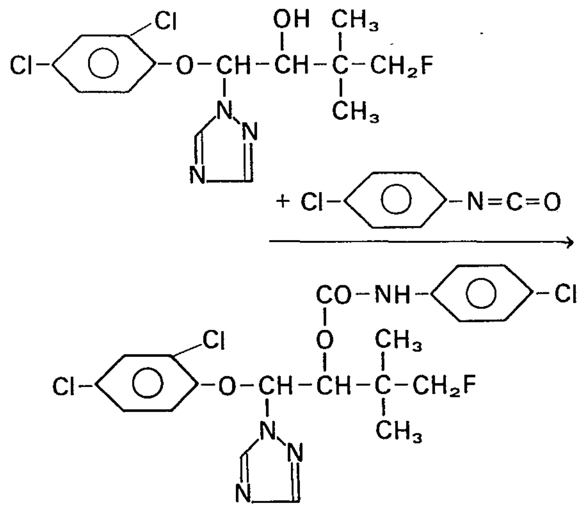

Umsetzungen von 1-Triazolyl-2-hydroxybutanderivaten der allgemeinen Formel II mit einem Keten der allgemeinen Formel V lassen sich in entsprechender Weise formulieren (Verfahren c).

Die für alle Verfahrensvarianten als Ausgangsstoffe zu verwendenden 1-Triazolyl-2-hydroxybutanderivate sind durch die allgemeine Formel II allgemein definiert. In dieser Formel stehen Az, X, Z und der Index n vorzugsweise für diejenigen Reste, die bereits im Zusammenhang mit der Beschreibung der erfindungsgemässen Stoffe der allgemeinen Formel I, vorzugsweise für diese Substituenten, genannt wurden.

Die 1-Triazolyl-2-hydroxybutanderivate der allgemeinen Formel II sind Gegenstand einer eigenen älteren Anmeldung (vergleiche DE-OS Nr. 2918894) und können hergestellt werden, indem man Halogenätherketone der allgemeinen Formel VII

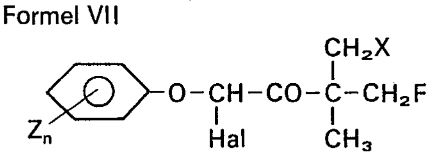 (VII)

in welcher

X, Z und n die oben angegebene Bedeutung haben, und

Hal für Halogen, vorzugsweise Chlor oder Brom steht,

mit 1,2,4-Triazol in Gegenwart eines Säurebindemittels, wie beispielsweise Natriumcarbonat oder ein Überschuss an Triazol, und gegebenenfalls in Gegenwart eines inerten organischen Lösungsmittels, wie beispielsweise Aceton oder Acetonitril, bei Temperaturen zwischen 60 und 120° C umsetzt und die so erhaltenen Ketoderivate nach bekannten Methoden reduziert, wie z.B. durch Umsetzung mit komplexen Hydriden, wie insbesondere Natriumborhydrid, gegebenenfalls in Gegenwart eines polaren organischen Lösungs-

mittels, wie beispielsweise Alkohole, bei Temperaturen zwischen 0 und 30° C; oder durch Umsetzung mit Aluminiumisopropylat in Gegenwart eines inerten organischen Lösungsmittels, wie beispielsweise Isopropanol, bei Temperaturen zwischen 20 und 120° C. Die Aufarbeitung erfolgt in üblicher Weise.

Die Halogenätherketone der allgemeinen Formel VII sind noch nicht bekannt. Sie sind jedoch ebenfalls Gegenstand der oben genannten eigenen älteren Anmeldung und können nach bekannten Verfahren (vgl. z.B. DE-OS 2632603) erhalten werden, indem man z.B. bekannte Phenole der allgemeinen Formel VIII

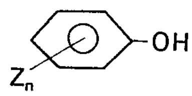

(VIII)

in welcher

Z und n die oben angegebene Bedeutung haben,

mit einem Halogenketon der allgemeinen Formel IX

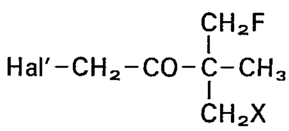

(IX)

in welcher

X die oben angegebene Bedeutung hat, und

Hal' für Chlor oder Brom steht,

umsetzt. Das noch verbliebene aktive Wasserstoffatom wird anschliessend in üblicher Weise gegen Halogen ausgetauscht (vgl. auch die Herstellungsbeispiele).

Die Halogenketone der allgemeinen Formel IX sind ebenfalls Gegenstand der oben genannten eigenen älteren Anmeldung. Sie können auf allgemein übliche und bekannte Weise erhalten werden, indem man Fluorderivate des 3,3-Dimethylbutan-2-ons der allgemeinen Formel X

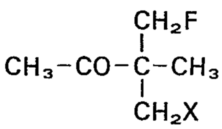

(X)

in welcher

X die oben angegebene Bedeutung hat,

mit Chlor und Brom in Gegenwart eines inerten organischen Lösungsmittels, wie beispielsweise Äther oder chlorierte Kohlenwasserstoffe, bei Raumtemperatur versetzt (vgl. auch die Herstellungsbeispiele), oder mit üblichen Chlorierungsmitteln, wie beispielsweise Sulfurylchlorid bei 20 bis 60° C umsetzt.

Die Fluorderivate des 3,3-Dimethylbutan-2-ons der allgemeinen Formel X sind Gegenstand einer eigenen älteren Patentanmeldung (vgl. DE-OS Nr. 2843767). Man erhält die Fluorderivate des 3,3-Dimethylbutan-2-ons der allgemeinen Formel X, wenn man Sulfonsäureester der allgemeinen Formel XI

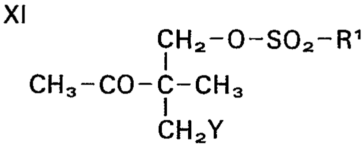

(XI)

in welcher

R' für Alkyl mit 1 bis 4 Kohlenstoffatomen, insbesondere Methyl, oder Aryl mit 6 bis 12 Kohlenstoffatomen, insbesondere Phenyl oder Tolyl, steht, und

Y für Wasserstoff oder die Gruppe $-O-SO_2-R^1$ steht,

mit Metallfluoriden, wie beispielsweise Natrium- und Kaliumfluorid, in Gegenwart eines polaren organischen Lösungsmittels, wie beispielsweise Di-, Tri- oder Tetraäthylenglykol, bei Temperaturen zwischen 80 und 250° C umsetzt (vgl. auch die Herstellungsbeispiele).

Die Sulfonsäureester der allgemeinen Formel XI sind teilweise bekannt [J. Org. Chem. *35*, 2391 (1970)]. Die noch nicht beschriebenen Verbindungen können nach literaturbekannten Verfahren aus den entsprechenden Hydroxybutanonen und Sulfochloriden in Gegenwart von Basen hergestellt werden (s. z.B. Houben-Weyl, „Methoden der Org. Chemie", Bd. IX, S. 388 und 663, sowie die Angaben bei den Herstellungsbeispielen).

Im einzelnen seien als Beispiele für die Ausgangsstoffe der allgemeinen Formel II genannt (Az steht dabei sowohl für 1,2,4-Triazol-1-yl als auch für 1,2,4-Triazol-4-yl):

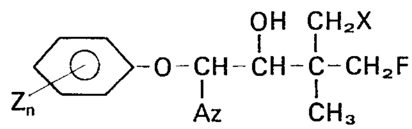

(II)

| $Z_n$ | X | $Z_n$ | X |
|---|---|---|---|
| – | H | – | F |
| 2–Cl | H | 2–Cl | F |
| 3–Cl | H | 3–Cl | F |
| 4–Cl | H | 4–Cl | F |
| 2–F | H | 2–F | F |
| 3–F | H | 3–F | F |
| 4–F | H | 4–F | F |
| 2–Br | H | 2–Br | F |
| 3–Br | H | 3–Br | F |
| 4–Br | H | 4–Br | F |
| 2,4–$Cl_2$ | H | 2,4–$Cl_2$ | F |
| 2–$CH_3$ | H | 2–$CH_3$ | F |
| 4–$CH_3$ | H | 4–$CH_3$ | F |
| 2–Cl, 4–$CH_3$ | H | 2–Cl, 4–$CH_3$ | F |
| 4–Cl, 2–$CH_3$ | H | 4–Cl, 2–$CH_3$ | F |
| 4–J | H | 4–J | F |
| 4–CN | H | 4–CN | F |
| 2–$NO_2$ | H | 2–$NO_2$ | F |
| 4–$COOCH_3$ | H | 4–$COOCH_3$ | F |
| 4–$COOC_2H_5$ | H | 4–$COOC_2H_5$ | F |

| $Z_n$ | X | $Z_n$ | X |
|---|---|---|---|
| 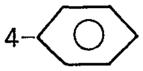 | H | 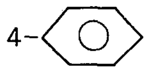 | F |
| 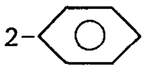 | H | 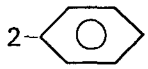 | F |
| 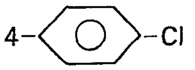 | H | 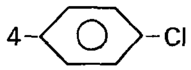 | F |

Die ausserdem für die Verfahrensvariante a als Ausgangsstoffe zu verwendenden Säurehalogenide sind durch die allgemeinen Formel III allgemein definiert. In dieser Formel steht R vorzugsweise für diejenigen Reste, die bereits im Zusammenhang mit der Beschreibung der erfindungsgemässen Stoffe der allgemeinen Formel I vorzugsweise für diesen Substituenten genannt wurden.

Die Säurehalogenide der allgemeinen Formel III sind bekannt oder lassen sich nach üblichen Verfahren herstellen; so z.B. durch Umsetzung von Carbonsäuren bzw. deren Alkalisalzen mit Säurehalogeniden des Phosphors oder Schwefels. Diese Methoden sind aus den allgemeinen Lehrbüchern der organischen Chemie bekannt.

Die weiterhin für die Verfahrensvariante b als Ausgangsstoffe zu verwendenden Säureanhydride sind durch die allgemeine Formel IV allgemein definiert. In dieser Formel steht R vorzugsweise für diejenigen Reste, die bereits im Zusammenhang mit der Beschreibung der erfindungsgemässen Stoffe der allgemeinen Formel I, vorzugsweise für diesen Substituenten, genannt wurden.

Die Säureanhydride der allgemeinen Formel IV sind bekannt oder lassen sich nach bekannten Verfahren herstellen; so z.B. durch Einwirkung von Säurechloriden auf die Alkalisalze der Carbonsäuren. Diese Verfahren sind allgemein bekannt.

Die ausserdem für die Verfahrensvariante c als Ausgangsstoffe zu verwendenden Ketene sind durch die allgemeine Formel V allgemein definiert.

Die Ketene der allgemeinen Formel V sind bekannt oder lassen sich nach bekannten Verfahren herstellen, so z.B. durch Thermolyse von Ketonen oder durch Dehydratisierung von Carbonsäuren (vgl. Houben-Weyl, „Methoden der organischen Chemie", Bd. 7/4, Georg-Thieme-Verlag).

Die weiterhin für die Verfahrensvariante d als Ausgangsstoffe zu verwendenden Isocyanate sind durch die allgemeine Formel VI allgemein definiert.

Die Isocyanate der allgemeinen Formel VI sind bekannt oder lassen sich nach allgemein üblichen und bekannten Verfahren herstellen; so z.B. durch Umsetzen von Aminen mit Phosgen und anschliessendem Erhitzen.

Als Lösungsmittel kommen für die Umsetzung gemäss Verfahrensvariante a vorzugsweise alle inerten organischen Lösungsmittel in Frage. Hierzu gehören vorzugsweise Nitrile, wie Propionitril, insbesondere Acetonitril; Äther, wie Tetrahydrofuran oder Dioxan; Ester, wie Essigsäureäthylester; aromatische Kohlenwasserstoffe, wie Benzol oder Toluol und halogenierte Kohlenwasserstoffe, wie Methylenchlorid, Tetrachlorkohlenstoff oder Chloroform. Der Einfachheit halber kann das eingesetzte Säurechlorid auch als Lösungsmittel verwendet werden, somit ein entsprechender Überschuss notwendig wird.

Die Reaktionstemperaturen können bei der Durchführung der Verfahrensvariante a in einem grösseren Bereich variiert werden. Im allgemeinen arbeitet man zwischen 0 und 100° C, vorzugsweise zwischen 20 und 85° C. Bei der Anwesenheit eines Lösungsmittels wird gegebenenfalls beim Siedepunkt des jeweiligen Lösungsmittels gearbeitet.

Das erfindungsgemässe Verfahren a kann gegebenenfalls in Gegenwart von Säurebindern (Halogenwasserstoffakzeptoren) durchgeführt werden; als solche können alle üblichen Säurebindungsmittel verwendet werden. Hierzu gehören organische Basen, vorzugsweise tertiäre Amine, wie z.B. Triäthylamin; ferner anorganische Basen, wie z.B. Alkalihydroxide und Alkalicarbonate.

Bei der Durchführung der Verfahrensvariante a arbeitet man vorzugsweise in molaren Mengen. Die Verbindungen der allgemeinen Formel I fallen in Form ihrer Hydrohalogenide an und können als solche isoliert werden, indem man sie durch Zugabe eines organischen Solvens, z.B. Hexan ausfällt, absaugt und gegebenenfalls durch Umkristallisation reinigt. Die Verbindungen der allgemeinen Formel I können auch in Form ihrer freien Base isoliert werden, indem man das Reaktionsgemisch mit wässeriger Natriumhydrogencarbonatlösung versetzt und die Base nach üblichen Methoden isoliert.

Als Verdünnungsmittel kommen für die Umsetzung gemäss Verfahrensvariante b vorzugsweise alle inerten organischen Lösungsmittel in Frage. Hierzu gehören vorzugsweise die bei der Verfahrensvariante a aufgezählten Solvenzien sowie die jeweils verwendeten Säureanhydride der allgemeinen Formel IV.

Als Katalysatoren können bei der Verfahrensvariante b vorzugsweise alle üblichen sauren und basischen Katalysatoren verwendet werden, wie z.B. Schwefelsäure, Chlorwasserstoff, Bromwasserstoff, Bortrifluorid, Zinkchlorid, Natriumacetat, Natriumbenzoat, Natriumcarbonat, Calciumoxid, Magnesiumoxid.

Die Reaktionstemperaturen können bei der Durchführung der Verfahrensvariante b in einem grösseren Bereich variiert werden. Im allgemeinen arbeitet man zwischen 0 und 150° C, vorzugsweise zwischen 80 und 120° C.

Bei der Durchführung der Verfahrensvariante b arbeitet man vorzugsweise in molaren Mengen. Der Einfachheit halber kann man das eingesetzte Säureanhydrid der allgemeinen Formel IV auch als Lösungsmittel verwenden, womit ein entsprechender Überschuss erforderlich wird. Die Isolierung der Verbindungen der allgemeinen Formel I erfolgt in üblicher Weise.

Als Verdünnungsmittel kommen für die Umsetzung gemäss Verfahrensvariante c vorzugsweise alle inerten organischen Lösungsmittel in Frage.

Hierzu gehören vorzugsweise die bei der Verfahrensvariante a aufgeführten Solvenzien.

Als Katalysatoren können bei der Verfahrensvariante c vorzugsweise alle üblichen sauren und basischen Katalysatoren verwendet werden. Hierzu gehören vorzugsweise die bei der Verfahrensvariante b aufgezählten Stoffe.

Die Reaktionstemperaturen können bei der Durchführung der Verfahrensvariante c in einem bestimmten Bereich variiert werden. Im allgemeinen arbeitet man zwischen –10 und 70° C, vorzugsweise zwischen 0 und 40° C.

Bei der Durchführung der Verfahrensvariante c arbeitet man vorzugsweise in molaren Mengen. Die Isolierung der Verbindungen der allgemeinen Formel I erfolgt nach üblichen Methoden.

Als Verdünnungsmittel kommen für die Umsetzung gemäss Verfahrensvariante d vorzugsweise alle inerten organischen Lösungsmittel in Frage. Hierzu gehören vorzugsweise die bei der Verfahrensvariante a aufgezählten Solvenzien.

Als Katalysatoren können beim Verfahren d vorzugsweise verwendet werden: tertiäre Basen, wie Triäthylamin und Pyridin oder zinnorganische Verbindungen, wie Dibutylzinndilaurat.

Die Reaktionstemperaturen können bei der Durchführung der Verfahrensvariante d in einem grösseren Bereich variiert werden. Im allgemeinen arbeitet man zwischen 0 und 100° C, vorzugsweise zwischen 20 und 40° C.

Bei der Durchführung der Verfahrensvariante d arbeitet man vorzugsweise in molaren Mengen. Zur Isolierung der Verbindungen der allgemeinen Formel I wird das Lösungsmittel abdestilliert und der Rückstand nach üblichen Methoden aufgearbeitet.

Zur Herstellung von Säureadditionssalzen der Verbindungen der allgemeinen Formel I kommen alle physiologisch verträglichen Säuren in Frage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Milchsäure, sowie Sulfonsäuren, wie z.B. p-Toluolsulfonsäure und 1,5-Naphthalindisulfonsäure.

Die Salze der Verbindungen der allgemeinen Formel I können in einfacher Weise nach üblichen Salzbildungsmethoden, z.B. durch Lösen einer Verbindung der allgemeinen Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalzkomplexen der Verbindungen der allgemeinen Formel I kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der allgemeinen Formel I können in einfacher Weise nach üblichen Verfahren erhalten werden, so z.B. durch Lösen des Metallsalzes in Alkohol, z.B. Äthanol, und Hinzufügen zur Verbindung der allgemeinen Formel I. Man kann Metallsalzkomplexe in bekannter Weise, z.B. durch Abfiltrieren, isolieren und gegebenenfalls durch Umkristallisieren reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromyzetes, Comyzetes, Chytridiomyzetes, Zygomyzetes, Ascomyzetes, Basidiomyzetes, Deuteromyzetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung solcher Pilze eingesetzt werden, die echte Mehltauerkrankungen hervorrufen; so zur Bekämpfung von *Podosphaera*-Arten, wie z.B. gegen den Erreger des Apfelmehltaus *(Podosphaera leucotricha)*, von *Erysiphe*-Arten, wie z.B. gegen den Erreger des Gurkenmehltaus *(Erysiphe cichoracearum)* oder des Gerstenmehltaus bzw. des Getreidemehltaus *(Erysiphe graminis)*; sowie zur Bekämpfung anderer Getreidekrankheiten, wie Getreiderost. Besonders hervorzuheben ist, dass die erfindungsgemässen Wirkstoffe nicht nur eine protektive Wirkung entfalten, sondern teilweise auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebelformulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glykol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylenfettsäureester, Polyoxyäthylenfettalkoholäther, z.B. Alkylarylpolyglykoläther, Alkylsulfonate, Alkylsulfate, Arylsulfonate sowie Eiweisshydrolysate; als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azolmetallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trokkenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.%, vorzugsweise von 0,0001 bis 0,2%, am Wirkungsort erforderlich.

*Herstellungsbeispiele*

*Beispiel 1*

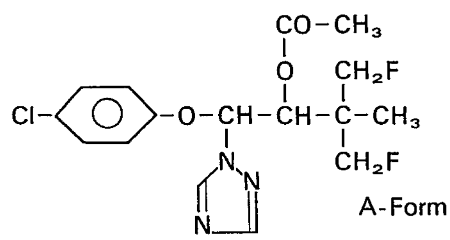

A-Form* = Bsp. 1a

B-Form* = Bsp. 1b

(Verfahren b)

112 g (0,328 mol) 3,3-Bisfluormethyl-1-(4-chlorphenoxy)-1-(1,2,4-triazol-1-yl)butan-2-ol (als Diastereomerengemisch der reinen Formen A und B)* werden in 500 ml Acetanhydrid gelöst. Man lässt 16 h bei 100°C rühren, destilliert das überschüssige Acetanhydrid im Vakuum ab und nimmt den Rückstand in 600 ml Methylenchlorid auf. Die organische Phase wird zweimal mit je 1,5 l Wasser gewaschen, über Natriumsulfat getrocknet und eingeengt. Der ölige Rückstand wird in Diisopropyläther fraktioniert kristallisiert. Man erhält 48 g (38% der Theorie) 2-Acetoxy-3,3-bisfluoromethyl-1-(4-chlorphenoxy)-1-(1,2,4-triazol-1-yl)butan als A-Form vom Schmelzpunkt 130-133° C und 20,5 g (16% der Theorie) 2-Acetoxy-3,3-bisfluormethyl-1-(4-chlorphenoxy)-1-(1,2,4-triazol-1-yl)butan als B-Form vom Schmelzpunkt 98° C.

---

* A- und B-Form = jeweils eine der beiden möglichen geometrischen Isomeren.

*Herstellung der Vorstufen*

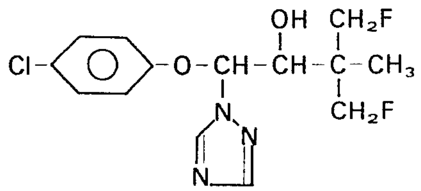

130,5 g (0,395 mol) 3,3-Bisfluormethyl-1-(4-chlorphenoxy)-1- (1,2,4-triazol-1-yl)- 2-butanon werden in 800 ml Methanol gelöst und portionsweise mit 21 g (0,55 mol) Natriumborhydrid versetzt. Man lässt die Reaktionslösung 15 h nachrühren und stellt sie dann mit konzentrierter Salzsäure auf einen pH-Wert von 3 ein. Man lässt 2 h nachrühren. Nach Abdestillieren des Lösungsmittels im Vakuum wird der Rückstand mit Wasser/Natriumhydrogencarbonat verstzt und mit 600 ml Methylenchlorid ausgeschüttelt. Die vereinigten organischen Phasen werden über Natriumsulfat getrocknet und im Vakuum eingeengt. Der Rückstand wird mit Diisopropyläther kristallisiert. Man erhält 112 g (86% der Theorie) 3,3-Bisfluormethyl-1- (1,2,4- triazol-1- yl)butan-2-ol vom Schmelzpunkt 97-102° C.

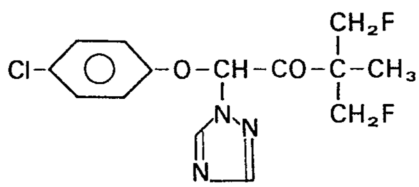

190 g (0,557 mol) 3,3-Bisfluormethyl-1-brom-1-(4-chlorphenoxy)butan-2-on und 90 g (1,3 mol) Triazol werden in 800 ml Acetonitril vorgelegt, 5 h bei 50° C erhitzt, das Lösungsmittel im Wasserstrahlvakuum abdestilliert, der Rückstand in 1 l Methylenchlorid aufgenommen und zweimal mit je 1000 ml Wasser gewaschen, die organische Phase wird über Natriumsulfat getrocknet und das Lösungsmittel abdestilliert. Der Rückstand wird in 500 ml Diisopropyläther aufgenommen und der Niederschlag abgesaugt. Die Mutterlauge wird destilliert. Man erhält 130,5 g (71% der Theorie) 3,3-Bisfluormethyl-1- (4-chlorphenoxy)- 1-(1,2,4-triazol-1-yl)-butan-2-on vom Siedepunkt 160-166° C/0,2 mmHg-Säule.

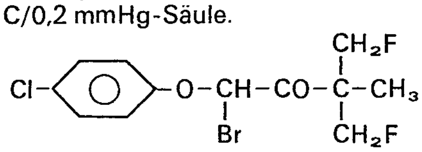

166 g (0,632 mol) 3,3-Bisfluormethyl-1-(4-chlorphenoxy)butan-2-on werden in 500 ml Methylenchlorid gelöst und bei 20 bis 30° C unter Rühren und Kühlen tropfenweise mit 100 g (0,625 mol) Brom versetzt. Es wird 2 h bei 20° C nachgerührt. Nach Abdestillieren des Lösungsmittels im Vakuum wird der Rückstand aus Petroläther kristallisiert. Man erhält 190 g (88% der Theorie) 3,3-Bisfluormethyl-1-brom-1-(4-chlorphenoxy)butan-2-on vom Schmelzpunkt 54-57° C.

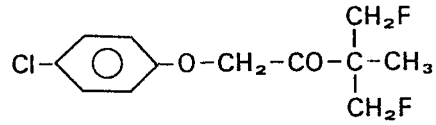

Zu einer gerührten Mischung aus 102 g (0,79 mol) p-Chlorphenol und 110 g (0,79 mol) gepulvertem Kaliumcarbonat in 500 ml Aceton werden bei 20 bis 30° C 171,2 g (0,79 mol) 3,3-Bisfluormethyl-1-brombutan-2-on zugetropft. Es wird 4 h bei 40° C nachgerührt, das anorganische Salz abfiltriert und das Filtrat eingeengt. Der Rückstand wird im Hochvakuum destilliert. Man erhält 190,5 g (90% der Theorie) 3,3-Bisfluormethyl-1-(4-chlorphenoxy)butan-2-on vom Siedepunkt 113-117° C/0,1 mmHg-Säule.

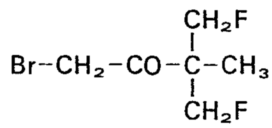

In eine Mischung aus 757,0 g (5,58 mol) 3,3-Bisfluormethyl-2-butanon und 4,5 l Methylenchlorid werden bei 20 bis 30° C unter Kühlen und Rühren 903 g Brom langsam eingetropft. Die gelbliche Lösung wird noch 1 h bei 20° C nachgerührt. Nach Abdestillieren des Lösungsmittels wird der Rückstand im Vakuum destilliert. Man erhält 1030 g (86% der Theorie) 3,3-Bisfluormethyl-1-brombutan-2-on vom Siedepunkt 49-53° C/ 0,15 mmHg-Säule.

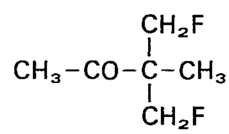

In einem Dreihalskolben mit Rührer, Tropftrichter und Liebigkühler Vorlage werden 400 ml Tetraäthylenglykol und 46,4 g Kaliumfluorid (0,8 mol) vorgelegt und auf 170° C aufgeheizt. Man legt an den Vorstoss des Liebigkühlers ein Wasserstrahlvakuum (Druck ca. 20 bis 30 mbar) an. Dann werden während 45 min 57,6 g (0,2 mol) 2-Acetyl-2-methylpropan-1,3-diolbismethansulfat, gelöst in 100 ml Tetraäthylenglykol, zugetropft. Das entstehende 3,3-Bisfluormethylbutan-2-on wird während der Reaktion in die gekühlte Vorlage destilliert. Nach dem Zutropfen wird noch während 1 h bei 175° C weiter destilliert.

Das aufgefangene Destillat wird anschliessend redestilliert. Man erhält 14 g (ca. 51,5% der Theorie) 3,3-Bisfluormethylbutan-2-on vom Siedepunkt 43-46° C/12 mmHg-Säule.

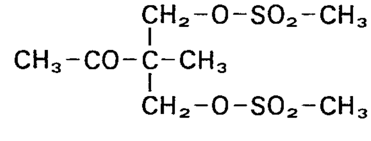

66 g (0,5 mol) 3-Oxa-2,2-bis(hydroxymethyl)-butan (zur Herstellung vgl. „Beilstein", *H 1* E III 3306, IV 4132 und „J. Chem. Soc.", London, *1932*, 2671) werden in 300 ml 1,2-Dichloräthan gelöst, 114,5 g (1 mol) Methansulfonsäurechlorid zugetropft und bei 0 bis 5° C 158 g (2 mol) Pyridin

zugetropft. Man lässt 15 h bei Raumtemperatur nachrühren und gibt dann den Ansatz auf 600 ml Eiswasser und 100 ml konzentrierter Salzsäure. Dabei fällt ein Feststoff aus, der abgesaugt wird. Die wässerige Phase wird mit 1000 ml Methylenchlorid extrahiert; in der Methylenchloridphase wird der Feststoff gelöst, die organische Phase über Natriumsulfat getrocknet, das Lösungsmittel im Wasserstrahlvakuum abdestilliert und der Rückstand in 200 ml Äther suspendiert. Der Rückstand wird abgesaugt und mit 100 ml Äther gewaschen. Man erhält 100 g (ca. 70% der Theorie) 2-Acetyl- 2-methylpropan- 1,3-diolbismethansulfonat vom Schmelzpunkt 105-108° C.

*Beispiel 2*

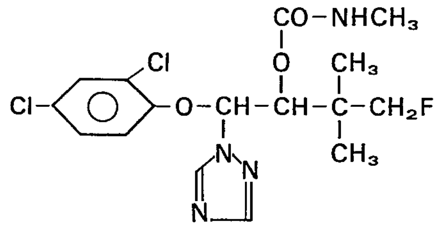

(Verfahren d)

8,4 g (0,024 mol) 1-(2,4-Dichlorphenoxy)-3,3-dimethyl-4-fluor-1-(1,2,4-triazol-1-yl)-butan-2-ol werden in 100 ml Dioxan gelöst und mit 2 ml Methylisocyanat sowie 3,5 ml Triäthylamin versetzt. Man erhitzt 10 h unter Rückfluss und engt durch Abdestillieren des Lösungsmittels im Vakuum ein. Der Rückstand wird in 50 ml Essigester erhitzt und filtriert. Man erhält 8 g (86% der Theorie) 1-(2,4-Dichlorphenoxy)-3,3- dimethyl-4-fluor-2-methylcarbamoyloxy-1-(1,2,4-triazol-1-yl)butan in Form farbloser Kristalle vom Schmelzpunkt 124-27° C.

*Herstellung der Vorstufen*

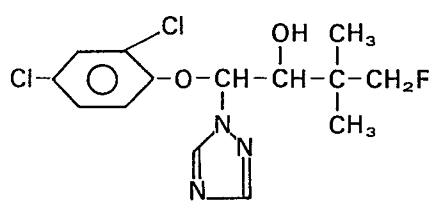

61,1 g (0,176 mol) 1-(2,4-Dichlorphenoxy)-3,3-dimethyl- 4-fluor-1- (1,2,4-triazol-1-yl)- 2-butanon werden in 250 ml Methanol gelöst und portionsweise mit 3 g (0,08 mol) Natriumborhydrid versetzt. Man lässt die Reaktionslösung 1 h nachrühren und stellt sie dann mit konzentrierter Salzsäure auf einen pH-Wert von 3 ein. Nach Abdestillieren des Lösungsmittels im Vakuum wird der Rückstand mit Wasser versetzt und mit Methylenchlorid ausgeschüttelt. Die vereinigten organischen Phasen werden über Natriumsulfat getrocknet und im Vakuum eingeengt. Der Rückstand wird mit Diethylether kristallisiert. Man erhält 44,4 g (72% der Theorie) 1-(2,4-Dichlorphenoxy)-3,3-dimethyl-4-fluor-1-(1,2,4-triazol-1-yl)-2-butanol vom Schmelzpunkt 98-100° C.

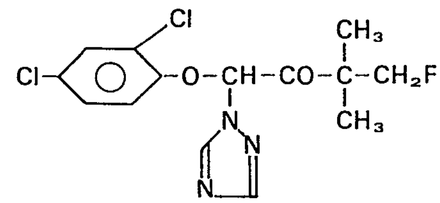

96,3 g (0,27 mol) 1-Brom-1-(2,4-dichlorphenoxy)-3,3 dimethyl-4-fluor-2-butanon werden in 200 ml Acetonitril gelöst und zu einer siedenden Lösung von 46 g (0,56 mol) 1,2,4-Triazol in 200 ml Acetonitril getropft. Nach 20stündigem Erhitzen unter Rückfluss wird das Lösungsmittel im Vakuum entfernt, der Rückstand mit Methylenchlorid aufgenommen, mehrmals mit Wasser gewaschen und die organische Phase über Natriumsulfat getrocknet. Nach Entfernen des Lösungsmittels im Vakuum erhält man 83 g (89% der Theorie) 1-(2,4-Dichlorphenoxy)-3,3-dimethyl-4-fluor-1-(1,2,4-triazol-1-yl)-2-butanon als Öl, das direkt weiter eingesetzt wird.

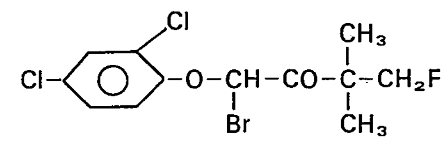

199,5 g (0,71 mol) 1-(2,4-Dichlorphenoxy)-3,3-dimethyl-4-fluor-2-butanon werden in 500 ml Chloroform gelöst und bei 20° C unter Rühren und Kühlen tropfenweise mit 114 g (0,71 mol) Brom versetzt. Es wird 2 h bei 20° C nachgerührt, vorsichtig mit 200 ml Wasser versetzt, die Chloroformphase mehrmals mit Eiswasser gewaschen und über Natriumsulfat getrocknet. Nach Abdestillieren des Lösungsmittels im Vakuum erhält man 205,2 g (78% der Theorie) 1-Brom-1- (2,4- Dichlorphenoxy)-3,3- dimethyl-4-fluor-2-butanon als Öl, das direkt weiter umgesetzt wird.

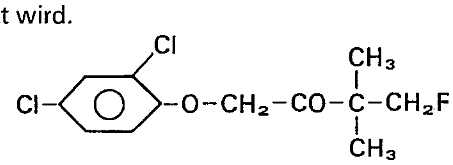

Zu einer gerührten Mischung aus 129 g (0,79 mol) 2,4-Dichlorphenol und 110 g (0,79 mol) gepulvertem Kaliumcarbonat in 500 ml Aceton werden unter Kühlen bei 20 bis 30° C 157 g (0,79 mol) 1-Brom-3,3-dimethyl-4-fluor-2-butanon zugetropft. Es wird 2 h bei 20° C nachgerührt, das anorganische Salz abfiltriert und das Filtrat eingeengt. Man erhält 199,3 g (90% der Theorie) 1-(2,4- Dichlorphenoxy)-3,3- dimethyl-4-fluor-2-butanon als Öl, das direkt weiter umgesetzt wird.

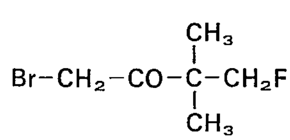

In eine Mischung aus 354 g (3 mol) 3,3-Dimethyl-4-fluor-2-butanon und 2000 ml Äther werden bei 20 bis 30° C unter Kühlen und Rühren

480 g Brom langsam eingetropft. Die gelbliche Lösung wird noch 1 h bei 20° C nachgerührt und anschliessend vorsichtig mit 500 ml Wasser versetzt. Die Ätherphase wird abgetrennt, mehrmals mit Wasser gewaschen und über Natriumsulfat getrocknet. Nach Abdestillieren des Lösungsmittels wird der Rückstand im Wasserstrahlvakuum destilliert. Man erhält 472 g (80% der Theorie) 1-Brom-3,3-dimethyl-4-fluor-2-butanon vom Siedepunkt 80-90° C/11 mmHg-Säule.

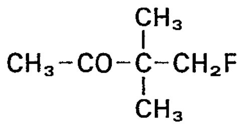

Zu der in einem Dreihals-Rührkolben mit absteigendem Kühler befindlichen Suspension von 23,2 g (0,4 mol) trockenem Kaliumfluorid in 400 ml destilliertem Tetraäthylenglykol werden bei 160° C und 20 mbar 38,8 g (0,2 mol) 2,2-Dimethyl-2-oxobutylmethansulfonat im Verlauf von 2 h zugetropft und 2 weitere Stunden nachgerührt. An einem absteigenden Kondensator und in einer nachgeschalteten Tiefkühlfalle wird das herausdestillierte Reaktionsprodukt kondensiert und gesammelt. Man erhält 20,9 g (89% der Theorie) 3,3-Dimethyl-4-fluor-2-butanon, vom Siedepunkt 130-134° C.

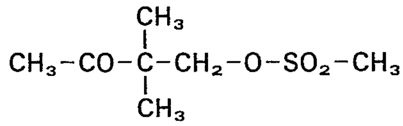

232 g (2 mol) 3,3-Dimethyl-4-hydroxy-2-butanon (z.Herstellung vgl. „Beilstein", *H 1*, E III, 3239, IV 4030 und „Bull. Soc. Chim.", France, *1954*, 2849) werden in 700 ml absolutem Pyridin bei 0 bis 5° C mit 229 g (2 mol) Methansulfochlorid umgesetzt. Nach 12 h Stehen bei 20° C wird mit Methylenchlorid verdünnt und mit Eiswasser ausgeschüttelt. Die organische Phase wird getrocknet, im Vakuum vom Lösungsmittel befreit und über eine Kolonne fraktioniert. Man erhält 332 g (86% der Theorie) 2,2-Dimethyl-3-oxobutylmethansulfonat vom Siedepunkt 106-120° C/0,12 mmHg-Säule.

In entsprechender Weise und gemäss den Verfahren a bis d werden die nachfolgenden Beispiele der allgemeinen Formel I

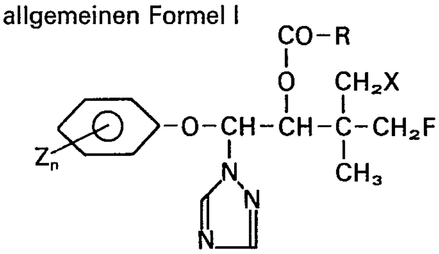

(I)

erhalten:

| Beispiel Nr. | $Z_n$ | R | X | Schmelzpunkt (°C) |
|---|---|---|---|---|
| 3 | 4−Cl | $-NHCH_3$ | H | Öl |
| 4 | 4−Cl | $-CH_3$ | H | Öl |
| 5 | 4−Cl | $-CH_2Cl$ | F | 123-25 (A-Form) |
| 6 | 4−Cl | $-CHCl_2$ | F | 214 (×½ NDS) |
| 7 | 4−Cl | $-NHCH_3$ | F | 144-50 (A-Form) |
| 8 | $2,4-Cl_2$ | $-NHCH_3$ | F | 131-34 (A-Form) |
| 9 | 4−F | $-CH_3$ | F | 136-38 (A-Form) |
| 10 | $2,4-Cl_2$ | $-CH_3$ | F | 123-25 (A-Form) |
| 11 | 4−F | $-CH_2Cl$ | F | 116-18 (A-Form) |
| 12 | $2,4-Cl_2$ | $-CH_2Cl$ | F | 89-91 (A-Form) |
| 13 | 4−F | $-CHCl_2$ | F | 63-66 (A-Form) |
| 14 | $2,4-Cl_2$ | $-CHCl_2$ | F | 98 (A-Form) |
| 15 | 4−F | $-NCH_3$ | F | 181-90 (A-Form) |
| 16 | 4−F | $-NH-CH_2OCH_3$ | F | 172 (A-Form) (×½ NDS) |
| 17 | 4- 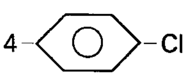 -Cl | $-CH_3$ | F | 83-87 |
| 18 | 4- 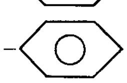 | $-NH-CH_3$ | F | 131-33 |
| 19 | 4- 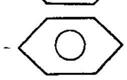 | $-NH-C_2H_5$ | F | 112-14 |

| Beispiel Nr. | $Z_n$ | R | X | Schmelzpunkt (°C) |
|---|---|---|---|---|
| 20 | 4- 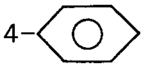 | $-NH-CH(CH_3)_2$ | F | 116-18 |
| 21 | $4-Cl$ | $-NH-CH(CH_3)_2$ | F | 110 |
| 22 | $2,4-Cl_2$ | $-NH-CH(CH_3)_2$ | F | 121-22 |
| 23 | $4-Cl$ | -NH- 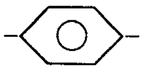 -Cl | F | 133 |
| 24 | $2,4-Cl_2$ | -NH- 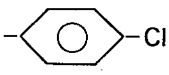 -Cl | F | 157-58 |
| 25 | $4-Cl$ | - 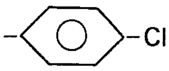 -Cl | F | 180 |
| 26 | 4- 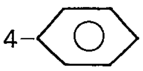 | $-NH-CH_2-O-CH_3$ | F | 180 (×½ NDS) |
| 27 | $4-Cl$ | $-NH-CH_2-O-CH_3$ | F | 180-83 (×½ NDS) |
| 28 | $2,4-Cl_2$ | $-NH-CH_2-O-CH_3$ | F | 210 (×½ NDS) |
| 29 | $4-Cl$ | $-CH_2-$ 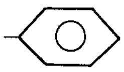 | F | 184-94 (×½ NDS) |
| 30 | $4-Cl$ | $-CH_2-O-C_2H_5$ | F | 181-88 (×½ NDS) |
| 31 | $4-Cl$ | 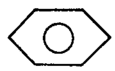 | F | 48-50 |
| 32 | $4-Cl$ | - 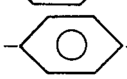 $-OCH_3$ | F | 187-89 (×½ NDS) |
| 33 | $4-Cl$ | $-CH_2-O-$ 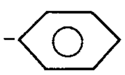 -Cl | F | 205-10 (×½ NDS) |
| 34 | $2,4-Cl_2$ | 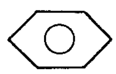 | F | 95-98 |
| 35 | $2,4-Cl_2$ | - 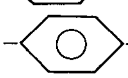 -Cl | F | 212-18 (×½ NDS) |
| 36 | $2,4-Cl_2$ | $-CH_2CH_2CH_2Cl$ | F | 212-18 (×½ NDS) |
| 37 | $4-Cl$ | $-CH_2CH_2CH_2Cl$ | F | 78-81 |
| 38 | $2,4-Cl_2$ | - 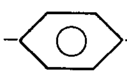 $-OCH_3$ | F | 185-90 (×½ NDS) |
| 39 | $4-Cl$ | $-C(CH_3)=CH_2$ | F | 191-98 (×½ NDS) |
| 40 | $2,4-Cl_2$ | - 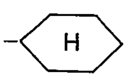 | F | 91-94 (A-Form) |
| 41 | $4-Cl$ | - 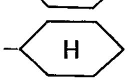 | F | 173 (A-Form) |
| 42 | $2,4-Cl_2$ | $CN_2-$ 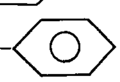 | F | 199-203 (×½ NDS) |
| 43 | $2,4-Cl_2$ | $CH_3$ | H | 145-8 |
| 44 | $4-Cl$ | $CH_2Cl$ | H | 104-120 |
| 45 | $4-Cl$ | $CHCl_2$ | H | Kristallbrei |

*Anwendungsbeispiele*

In den nachfolgenden Beispielen werden die nachstehend angegebenen Verbindungen als Vergleichssubstanzen eingesetzt:

(A) 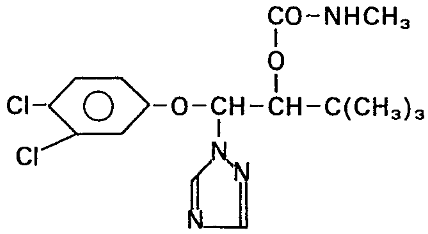

(B) 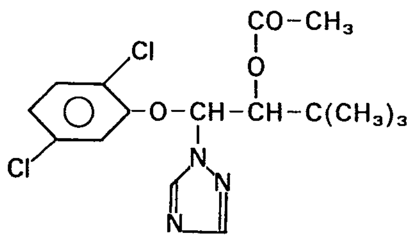

(C) 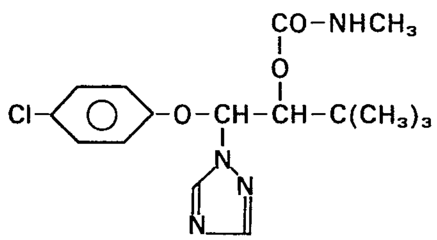

*Beispiel A*

Sprossbehandlungs-Test / Getreidemehltau / Protektiv (blattzerstörende Mykose)

Zur Herstellung einer zweckmässigen Wirkstoffzubereitung nimmt man 0,25 Gewichtsteile Wirkstoff in 25 Gewichtsteilen Dimethylformamid und 0,06 Gewichtsteilen Emulgator (Alkylarylpolyglykoläther) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von *Erysiphe graminis var.hordei.*

Nach 6 d Verweilzeit der Pflanzen bei einer Temperatur von 21-22° C und einer Luftfeuchtigkeit von 80-90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Bei diesem Test zeigen z.B. die folgenden Verbindungen eine sehr gute Wirkung, die derjenigen der aus dem Stand der Technik bekannten Verbindung A überlegen ist:

Verbindungen gemäss Herstellungsbeispielen 1a, 3, 4, 2 und 1b.

*Beispiel B*

Gerstenmehltau-Test *(Erysiphe graminis var.hordei)* / systemisch (pilzliche Getreidesprosskrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3×12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 d nach der Aussaat, wenn die Gerstenpflanzen ihr erstes Blatt entfaltet haben, werden sie mit frischen Sporen von *Erysiphe graminis var.hordei* bestäubt und bei 21-22° C und 80-90% rel.Luftfeuchte und 16stündiger Belichtung weiter kultiviert. Innerhalb von 6 d bilden sich an den Blättern die typischen Mehltaupusteln aus.

Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Bei diesem Test zeigen z.B. die folgenden Verbindungen eine sehr gute Wirkung, die derjenigen der aus dem Stand der Technik bekannten Verbindung B überlegen ist:

Verbindungen gemäss Herstellungsbeispielen 1a und 4,

*Beispiel C*

Sprossbehandlungs-Test / Getreiderost / protektiv (blattzerstörende Mykose)

Zur Herstellung einer zweckmässigen Wirkstoffzubereitung nimmt man 0,25 Gewichtsteile Wirkstoff in 25 Gewichtsteilen Dimethylformamid und 0,06 Gewichtsteilen Emulgator Alkylarylpolyglykoläther auf und gibt 975 Gewichtsteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von *Puccinia recondita* in 0,1%igem Wasseragar. Nach Antrocknen der Sporensuspension besprüht man die Weizenpflanzen mit der Wirkstoffzubereitung taufeucht und stellt sie zur Inkubation für 24 h bei etwa 20° C und einer 100%igen Luftfeuchtigkeit in ein Gewächshaus.

Nach 10 d Verweilzeit der Pflanzen bei einer Temperatur von 20° C und einer Luftfeuchtigkeit von 80-90% wertet man den Besatz der Pflanzen mit Rostpusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Rostbefall ist.

In diesem Test zeigen z.B. die folgenden Verbindungen eine sehr gute Wirkung, die derjenigen der aus dem Stand der Technik bekannten Verbindung B überlegen ist:

Verbindung gemäss Herstellungsbeispielen 1a und 4.

*Beispiel D*

*Erysiphe*-Test (Gurken)/Protektiv

| | |
|---|---|
| Lösungsmittel: | 4,7 Gewichtsteile Aceton |
| Emulgator: | 0,3 Gewichtsteile Alkylaryl-polyglykoläther |
| Wasser: | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 h im Gewächshaus. Dann werden sie zur Inokulation mit Konidien des Pilzes *Erysiphe cichoracearum* bestäubt. Die Pflanzen werden anschliessend bei 23 bis 24° C und bei einer relativen Luftfeuchtigkeit von ca. 75% im Gewächshaus aufgestellt.

Nach 12 h wird der Befall der Gurkenpflanzen bestimmt. Die erhaltenen Boniturwerte werden in Prozent Befall umgerechnet. 0% bedeutet keinen Befall, 100% bedeutet, dass die Pflanzen vollständig befallen sind.

Bei diesem Test zeigen z.B. folgende Verbindungen eine sehr gute Wirkung, die derjenigen der aus dem Stand der Technik bekannten Verbindung C überlegen ist:

Verbindung gemäss Herstellungsbeispiel 1a.

*Beispiel E*

*Podosphaera*-Test (Apfel) / protektiv

| | |
|---|---|
| Lösungsmittel: | 4,7 Gewichtsteile Aceton |
| Emulgator: | 0,3 Gewichsteile Alkylaryl-glykoläther |
| Wasser: | 95,0 Gewichtsteile |

Man vermischt die für die gewünschte Wirkstoffkonzentration in der spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 h bei 20° C und einer relativen Luftfeuchtigkeit von 70% im Gewächshaus. Anschliessend werden sie durch Bestäuben mit Konidien des Apfelmehltauerregers *(Podosphaera leucotricha)* inokuliert und in ein Gewächshaus mit einer Temperatur von 21 bis 23° C und einer relativen Luftfeuchtigkeit von 70% gebraucht.

10 d nach der Inokulation wird der Befall der Sämlinge bestimmt. Die erhaltenen Boniturwerte werden in Prozent Befall umgerechnet. 0% bedeutet keinen Befall, 100% bedeutet, dass die Pflanzen vollständig befallen sind.

In diesem Test zeigen z.B. folgende Verbindungen eine sehr gute Wirkung, die derjenigen der aus dem Stand der Technik bekannten Verbindung C deutlich überlegen ist:

Verbindungen gemäss Herstellungsbeispielen 1a, 4 und 1b.

## Patentansprüche

1. Acylierte Triazolyl-γ-fluorpinakolylderivate der allgemeinen Formel I

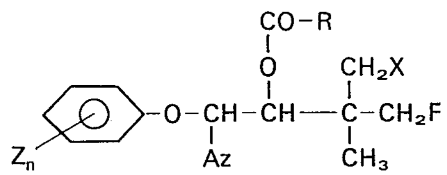

(I)

in welcher

Az für 1,2,4-Triazol-1-yl- oder 1,2,4-Triazol-4-yl steht,

R für Alkyl mit 1 bis 6, Alkenyl und Alkinyl mit jeweils 2 bis 4 Kohlenstoffatomen, Halogenalkyl mit 1 bis 3 Kohlenstoffatomen, Halogenalkyl mit 1 bis 3 Kohlenstoffatomen, Halogenalkyl mit 1 bis 3 Kohlenstoff- und 1 bis 5 Halogenatomen, Alkoxy und Alkoxyalkyl mit 1 bis 4 Kohlenstoffatomen in den Alkylteilen steht, ferner für Cyclohexyl steht, sodann für Phenyl, Phenylalkyl und Phenoxyalkyl mit jeweils bis zu 2 Kohlenstoffatomen im Alkylteil steht, wobei die drei letztgenannten Gruppen im Phenylteil substituiert sein können durch Halogen, Alkyl und Alkoxy mit jeweils 1 bis 2 Kohlenstoffatomen, weiterhin für Alkylamino mit 1 bis 12 Kohlenstoffatomen und für Dimethyl- oder Diäthylamino, Halogenalkylamino mit bis zu 4 Kohlenstoff- und 5 Halogenatomen steht, für Alkoxycarbonylamino und Alkoxyalkylamino mit jeweils 1 bis 4 Kohlenstoffatomen in jedem Alkylteil und endlich für Phenylamino steht, welches gegebenenfalls durch Halogen, Alkyl mit bis zu 4, Alkoxy und Alkylthio mit bis zu 2 Kohlenstoffatomen und durch Halogenalkyl mit bis zu 2 Kohlenstoff- und 5 Halogenatomen substituiert sein kann,

X für Wasserstoff oder Fluor steht,

Z für Halogen, Cyano, Nitro und Alkyl mit bis zu 4 Kohlenstoffatomen, für Cyclohexyl, für Halogenalkyl mit bis zu 2 Kohlenstoff- und 5 Halogenatomen, für Alkoxy und Alkylthio mit bis zu 2 und Alkoxycarbonyl mit insgesamt bis zu 5 Kohlenstoffatomen steht und weiterhin für Phenyl, Benzyl und Phenoxy steht, wobei die drei letztgenannten Reste gegebenenfalls durch Halogen substituiert sein können, und

n für ganze Zahlen von 0 bis 3 steht, und deren physiologisch verträglichen Säureadditionssalze und Metallsalzkomplexe.

2. Verfahren zur Herstellung von acylierten Triazolyl-γ-fluorpinakolylderivaten der allgemeinen Formel I

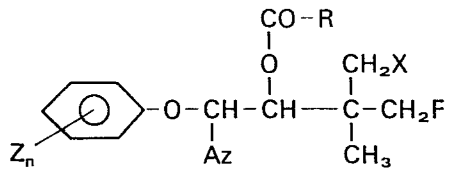

(I)

in welcher

Az für 1,2,4-Triazol-1-yl oder 1,2,4-Triazol-4-yl steht,

R für Alkyl mit 1 bis 6, Alkenyl und Alkinyl mit jeweils 2 bis 4 Kohlenstoffatomen, Halogenalkyl mit 1 bis 3 Kohlenstoff- und 1 bis 5 Halogenatomen, Alkoxy und Alkoxyalkyl mit 1 bis 4 Kohlenstoffatomen in den Alkylteilen steht, ferner für Cyclohexyl steht, sodann für Phenyl, Phenylalkyl und Phenoxyalkyl mit jeweils bis zu 2 Kohlenstoffatomen im Alkylteil steht, wobei die drei letztgenannten Gruppen im Phenylteil substituiert sein können durch Halogen, Alkyl und Alkoxy mit jeweils 1 bis 2 Kohlenstoffatomen, weiterhin für Alkylamino mit 1 bis 12 Kohlenstoffatomen und für Dimethyl- oder Diäthylamino, Halogenalkylamino mit zu 4 Kohlenstoff- und 5 Halogenatomen steht, für Alkoxycarbonylamino und Alkoxyalkylamino mit jeweils 1 bis 4 Kohlenstoffatomen in jedem Alkylteil und endlich für Phenylamino steht, welches gegebenenfalls durch Halogen, Alkyl mit bis zu 4, Alkoxy und Alkylthio mit bis zu 2 Kohlenstoffatomen und durch Halogenalkyl mit bis zu 2 Kohlenstoff- und 5 Halogenatomen substituiert sein kann,

X für Wasserstoff oder Fluor steht,

Z für Halogen, Cyano, Nitro und Alkyl mit bis zu 4 Kaholenstoffatomen, für Cyclohexyl, für Halogenalkyl mit bis zu 2 Kohlenstoff- und 5 Halogenatomen, für Alkoxy und Alkylthio mit bis zu 2 und Alkoxycarbonyl mit insgesamt bis zu 5 Kohlenstoffatomen steht und weiterhin für Phenyl, Benzyl und Phenoxy steht, wobei die drei letztgenannten Reste gegebenenfalls durch Halogen substituiert sein können, und

n für ganze Zahlen von 0 bis 3 steht,

und deren physiologisch verträglichen Säureadditionssalzen und Metallsalzkomplexen, dadurch gekennzeichnet, dass man 1-Triazolyl-2-hydroxy-butanderivate der allgemeinen Formel (II)

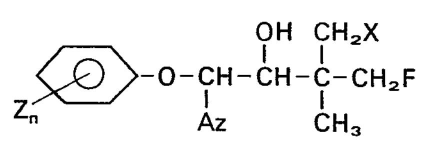

(II)

in welcher

Az, X, Z und n die oben angegebene Bedeutung haben,

a) mit Säurehalogeniden der allgemeinen Formel (III)

Hal−CO−R (III)

in welcher

R die oben angegebene Bedeutung hat und

Hal für Halogen steht,

gegebenenfalls in Gegenwart eines Lösungsmittels und gegebenenfalls in Gegenwart eines Säurebindemittels umsetzt, oder

b) mit Säureanhydriden der allgemeinen Formel (IV)

R−CO−O−CO−R (IV)

in welcher

R die oben angegebene Bedeutung hat,

in Gegenwart eines Lösungsmittels und gegebenenfalls in Gegenwart eines Katalysators umsetzt, oder

c) mit Ketenen der Allgemeinen Formel (V)

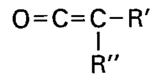

(V)

in welcher

R′ für Wasserstoff, für Alkyl mit bis zu 5 oder für Alkenyl oder Alkoxy mit bis zu 3 Kohlenstoffatomen steht, ferner für Phenyl steht, welches durch Halogen, Alkyl und Alkoxy mit bis zu 2 Kohlenstoffatomen substituiert sein kann, sodann für Halogenmethyl mit 1 bis 3 Fluor- und/oder Chloratomen steht, und schliesslich für Chlor oder Brom steht, und

R″ die unter R′ genannten Bedeutung annehmen kann, jedoch mit der Massgabe, dass die Summe der Kohlenstoffatome in den Alkyl-, Alkenyl- und Alkoxyresten für R′ und R″ gemeinsam um die Zahl 1 unter der Zahl derjenigen Kohlenstoffatome, die oben bei der R-Definition für die genannten Reste angegeben ist, bleiben muss, und dass höchstens einer der Reste R′ und R″ für gegebenenfalls wie angegeben substituiertes Phenyl oder Halogenmethyl stehen darf, in Gegenwart eines Lösungsmittels und gegebenenfalls in Gegenwart eines Katalysators umsetzt, oder

d) mit Isocyanaten der allgemeinen Formel (VI)

O=C=N−R‴ (VI)

in welcher

R‴ für Alkyl mit bis zu 12, für Halogenalkyl mit bis zu 4 Kohlenstoff- und bis zu 5 Halogenatomen steht, für Alkoxycarbonyl oder Alkoxyalkyl mit jeweils bis zu 4 Kohlenstoffatomen in jedem Alkylteil steht, oder für Phenyl steht, welches durch Halogen, durch Alkyl mit bis zu 4, Alkoxy und Alkylthio mit bis zu 2 Kohlenstoffatomen und durch Halogenalkyl mit bis zu 2 Kohlenstoff- und bis zu 5 Halogenatomen substituiert sein kann, in Gegenwart eines Lösungsmittels und gegebenenfalls in Gegenwart eines Katalysators umsetzt, und gegebenenfalls noch die nach Verfahren a bis b erhaltenen Verbindungen durch Umsetzung mit Säuren in die Salze bzw. durch Reaktion mit Metallsalzen in die entsprechenden Metallsalzkomplexe überführt.

3. Fungizide Mittel, gekennzeichnet durch einen Gehalt an mindestens einem acylierten Triazolyl-γ-fluorpinakolylderivat der allgemeinen Formel (I) in den Ansprüchen 1 und 2.

4. Verwendung von acylierten Triazol-γ-fluorpinakolylderivaten der allgemeinen Formel (I) in den Ansprüchen 1 und 2 zur Bekämpfung von Pilzen im Pflanzenschutz, soweit sie nicht die Human- und Veterinärmedizin betrifft.

5. Verfahren zur Herstellung von fungiziden Mitteln, dadurch gekennzeichnet, dass man acylierte Triazolyl-γ-fluorpinakolylderivate der allgemeinen Formel (I) in den Ansprüchen 1 und 2 mit Streckmitteln und/oder oberflächenaktiven Mitteln vermischt.

## Claims

1. Acylated triazolyl-γ-fluoropinacolyl derivatives of the general formula I

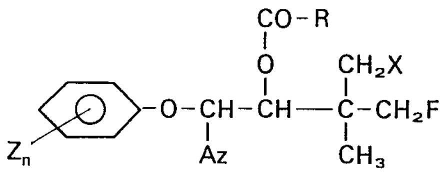

(I)

in which

Az represents 1,2,4-triazol-1-yl or 1,2,4-triazol-4-yl;

R represents alkyl with 1 to 6, alkenyl and alkinyl with in each case 2 to 4 carbon atoms, halogenoalkyl with 1 to 3 carbon and 1 to 5 halogen atoms, alkoxy and alkoxyalkyl with 1 to 4 carbon atoms in the alkyl parts, also cyclohexyl, furthermore phenyl, phenylalkyl and phenoxyalkyl with in each case up to 2 carbon atoms in the alkyl part, it being possible for the three last-mentioned groups to be substituted in the phenyl part by halogen, alkyl and alkoxy with in each case 1 to 2 carbon atoms, and dimethyl- or diethylamino, halogenolkylamino with up to 4 carbon and 5 halogen atoms, alkoxycarbonylamino and alkoxyalkylamino with in each case 1 to 4 carbon atoms in each alkyl part and finally phenylamino, which can optionally be substituted by halogen, alkyl with up to 4, alkoxy and alkylthio with up to 2 carbon atoms and by halogenoalkyl with up to 2 carbon and 5 halogen atoms;

X represents hydrogen or fluorine;

Z represents halogen, cyano, nitro and alkyl withup to 4 carbon atoms, cyclohexyl, halogenoalkyl with up to 2 carbon and 5 halogen atoms, alkoxy and alkylthio with up to 2 and alkoxycarbonyl with a total of up to 5 carbon atoms, and in addition phenyl, benzyl and phenoxy, it being possible for the three last-mentioned radicals to be optionally substituted by halogen, and

n represents integers from 0 to 3,

and physiologically acceptable acid addition salts and metal salt complexes thereof.

2. Process for the preparation of acylated triazolyl-γ-fluoropinacolyl derivatives of the general formula I

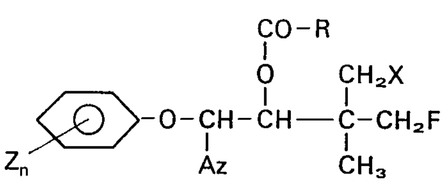

(I)

in which

Az represents 1,2,4-triazol-1-yl or 1,2,4-triazol-4-yl,

R represents alkyl with 1 to 6, alkenyl and alkinyl with in each case 2 to 4 carbon atoms, halogenoalkyl with 1 to 3 carbon and 1 to 5 halogen atoms, alkoxy and alkoxyalkyl with 1 to 4 carbon atoms in the alkyl parts, also cyclohexyl, furthermore phenyl, phenylalkyl and phenoxyalkyl with in each case up to 2 carbon atoms in the alkyl part, it being possible for the three last-mentioned groups to be substituted in the phenyl part by halogen, alkyl and alkoxy with in each case 1 to 2 carbon atoms, in addition alkylamino with 1 to 12 carbon atoms, and dimethyl- or diethylamino, halogenoalkylamino with up to 4 carbon and 5 halogen atoms, alkoxycarbonylamino and alkoxyalkylamino with in each case 1 to 4 carbon atoms in each alkyl part and finally phenylamino, which can optionally be substituted by halogen, alkyl with up to 4, alkoxy and alkylthio with up to 2 carbon atoms and by halogenoalkyl with up to 2 carbon and 5 halogen atoms;

X represents hydrogen or fluorine;

Z represents halogen, cyano, nitro and alkyl with up to 4 carbon atoms, cyclohexyl, halogenoalkyl with up to 2 carbon and 5 halogen atoms, alkoxy and alkylthio with up to 2 and alkoxycarbonyl with a total of up to 5 carbon atoms, and in addition phenyl, benzyl and phenoxy, it being possible for the three last-mentioned radicals to be optionally substituted by halogen, and

n represents integers from 0 to 3,

and physiologically acceptable acid addition salts and metal salt complexes thereof, characterised in that 1-triazolyl-2-hydroxy-butane derivatives of the general formula II

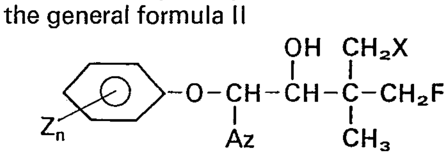

(II)

in which

Az, X, Z and n have the meaning indicated above,

(a) are reacted with acid halides of the general formula III

$$Hal-CO-R \quad (III)$$

in which

R has the meaning indicated above, and

Hal represents halogen,

if appropriate in the presence of a solvent and if appropriate in the presence of an acid-binding agent, or

(b) are reacted with acid anhydrides of the general formula IV

$$R-CO-O-CO-R \quad (IV)$$

in which

R has the meaning indicated above,

in the presence of a solvent and if appropriate in the presence of a catalyst, or

(c) are reacted with ketenes of the general formula V

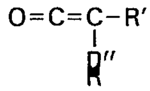

(V)

in which

R′ represents hydrogen, alkyl with up to 5 or alkenyl or alkoxy with up to 3 carbon atoms, also phenyl which can be substituted by halogen, alkyl and alkoxy with up to 2 carbon atoms, furthermore halogenomethyl with 1 to 3 fluorine and/or chlorine atoms, and finally chlorine or bromine, and

R″ can have the meanings mentioned under R′, but with the proviso that the sum of the carbon atoms in the alkyl, alkenyl and alkoxy radicals for R′ and R″ together must remain lower than the number of those carbon atoms which is indicated above, under the R definition, for the stated radicals, by the number 1, and that at most one of the radicals R′ and R″ may represent phenyl, optionally substituted as indicated, or halogenomethyl,

in the presence of a solvent and if appropriate in the presence of a catalyst, or

(d) are reacted with isocyanates of the general formula VI

$$O=C=N-R''' \qquad (VI)$$

in which

R‴ represents alkyl with up to 12, halogenoalkyl with up to 4 carbon and up to 5 halogen atoms, alkoxycarbonyl or alkoxyalkyl with in each case up to 4 carbon atoms in each alkyl part, or phenyl which can be substituted by halogen, by alkyl with up to 4, alkoxy and alkylthio with up to 2 carbon atoms and by halogenoalkyl with up to 2 carbon and up to 5 halogen atoms,

in the presence of a solvent and if appropriate in the presence of a catalyst, and the compounds obtained by processes (a) to (d) are optionally also converted into salts, by reaction with acids, or into the corresponding metal salt complexes, by reaction with metal salts.

3. Fungicidal agents, characterized in that they contain at least one acylated triazolyl-γ-fluoropinacolyl derivative of the general formula I in claims 1 and 2.

4. Use of acylated triazol-γ-fluoropinacolyl derivatives of the general formula I in claims 1 and 2 for the combating of fungi in plant protection, in as far as it does not relate to human and veterinary medicine.

5. Process for the preparation of fungicidal agents, characterized in that acylated triazolyl-γ-fluoropinacolyl derivatives of the general formula I in claims 1 and 2 are mixed with extenders and/or surface-active agents.

## Revendications

1. Dérivés de triazolyl-γ-fluoropinacolyle acylés de formule générale I

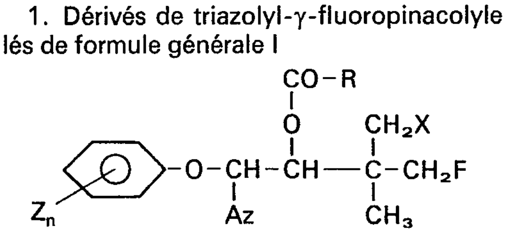

(I)

dans laquelle

Az est un groupe 1,2,4-triazol-1-yle ou 1,2,4-triazolyl-4-yle,

R représente un groupe alkyle ayant 1 à 6 atomes de carbone, alcényle et alcynyle ayant chacun 2 à 3 atomes de carbone, un groupe halogénalkyle ayant 1 à 3 atomes de carbone et 1 à 5 atomes d'halogènes, un groupe alkoxy et alkoxyalkyle ayant 1 à 4 atomes de carbone et 1 à 5 atomes d'halogènes, un groupe alkoxy et alkoxyalkyle ayant 1 à 4 atomes de carbone dans les parties alkyle, en outre le groupe cyclohexyle, le groupe phényle, un groupe phénylalkyle et un groupe phénoxyalkyle ayant chacun jusqu'à 2 atomes de carbone dans la partie alkyle, les trois groupes mentionnés en dernier lieu pouvant être substitués dans la partie phényle par un halogène, un radical alkyle et un radical alkoxy ayant chacun 1 ou 2 atomes de carbone, en outre un groupe alkylamino ayant 1 à 12 atomes de carbone et le groupe diméthyl- ou diéthylamino, un groupe halogénalkylamino ayant jusqu'à 4 atomes de carbone et jusqu'à 5 atomes d'halogènes, un groupe alkoxycarbonylamino et un groupe alkoxyalkylamino ayant chacun 1 à 4 atomes de carbone dans chaque partie alkyle, enfin un groupe phénylamino qui peut être substitué le cas échéant par un halogène, un radical alkyle ayant jusqu'à 4 atomes de carbone, un radical alkoxy et un radical alkylthio ayant jusqu'à 2 atomes de carbone et un radical halogénalkyle ayant jusqu'à 2 atomes de carbone et jusqu'à 5 atomes d'halogènes;

X est l'hydrogène ou le fluor,

Z est un halogène, un groupe cyano, nitro, ou alkyle ayant jusqu'à 4 atomes de carbone, le groupe cyclohexyle, un groupe halogénalkyle ayant jusqu'à 2 atomes de carbone et jusqu'à 5 atomes d'halogènes, un groupe alkoxy et un groupe alkylthio ayant jusqu'à 2 atomes de carbone et un groupe alkoxycarbonyle ayant au total jusqu'à 5 atomes de carbone, en outre les restes phényle, benzyle et phénoxy, les trois restes mentionnés en dernier lieu pouvant éventuellement être substitués par un halogène, et

n représente les nombres entiers de 0 à 3,

et leurs sels d'addition d'acides et leurs complexes de sels métalliques physiologiquement acceptables.

2. Procédé de production de dérivés de triazolyl-γ-fluoropinacolyle acylés de formule générale I

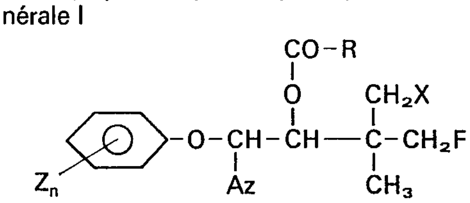

(I)

dans laquelle

Az est un groupe 1,2,4-triazol-1-yle ou 1,2,4-triazolyl-4-yle,

R représente un groupe alkyle ayant 1 à 6 atomes de carbone, alcényle et alcynyle ayant chacun 2 à 4 atomes de carbone, un groupe halogénalkyle ayant 1 à 3 atomes de carbone et 1

à 5 atomes d'halogènes, un groupe alkoxy et alkoxyalkyle ayant 1 à 4 atomes de carbone dans les parties alkyle, en outre le groupe cyclohexyle, le groupe phényle, un groupe phénylalkyle et un groupe phénoxyalkyle ayant chacun jusqu'à 2 atomes de carbone dans la partie alkyle, les trois groupes mentionnés dans la partie phényle par un halogène, un radical alkyle et un radical alkoxy ayant chacun 1 ou 2 atomes de carbone, en outre un groupe alkylamino ayant 1 à 12 atomes de carbone et le groupe diméthyl- ou diéthylamino, un groupe halogénalkylamino ayant jusqu'à 4 atomes de carbone et jusqu'à 5 atomes d'halogènes, un groupe alkoxycarbonylamino et un groupe alkoxyalkylamino ayant chacun 1 à 4 atomes de carbone dans chaque partie alkyle, enfin un groupe phénylamino qui peut être substitué le cas échéant par un halogène, un radical alkyle ayant jusqu'à 4 atomes de carbone, un radical alkoxy et un radical alkylthio ayant jusqu'à 2 atomes de carbone et un radical halogénalkyle ayant jusqu'à 2 atomes de carbone et jusqu'à 5 atomes d'halogènes;

X est l'hydrogène ou le fluor,

Z est un halogène, un groupe cyano, nitro ou alkyle ayant jusqu'à 4 atomes de carbone, le groupe cyclohexyle, un groupe halogenalkyle ayant jusqu'à 2 atomes d'halogènes, un groupe alkoxy et un groupe alkylthio ayant jusqu'à 2 atomes de carbone et un groupe alkoxycarbonyle ayant au total jusqu'à 5 atomes de carbone, en outre les restes phényle, benzyle et phénoxy, les trois restes mentionnés en dernier lieu pouvant éventuellement être substitués par un halogène, et

n représente les nombres entiers de 0 à 3,

et de leurs sels d'addition d'acides et de leurs complexes de sels métalliques physiologiquement acceptables, caractérisé en ce qu'on fait réagir des dérivés de 1-triazolyl-2-hydroxybutane de formule générale II

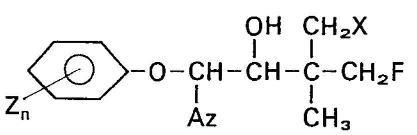 (II)

dans laquelle

Az, X, Z et n ont la définition indiquée ci-dessus,

a) avec des halogénures d'acides de formule générale III

$$Hal-Co-R \qquad (III)$$

dans laquelle

R a la définition indiquée ci-dessus, et

Hal désigne un halogène,

éventuellement en présence d'un solvant et en la présence éventuelle d'un accepteur d'acide, ou

b) avec des anhydrides d'acides de formule générale IV

$$R-CO-O-CO-R \qquad (IV)$$

dans laquelle

R a la définition indiquée ci-dessus, en présence d'un solvant et en la présence éventuelle d'un catalyseur, ou

c) avec des cétènes de formule générale V

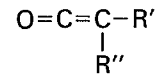 (V)

dans laquelle

R' représente l'hydrogène, un groupe alkyle ayant jusqu'à 5 atomes de carbone ou un groupe alcényle ou alkoxy ayant jusqu'à 3 atomes de carbone, en outre un groupe phényle qui peut être substitué par un halogène, un radical alkyle et un radical alkoxy ayant jusqu'à 2 atomes de carbone, ainsi qu'un groupe halogénométhyle ayant 1 à 3 atomes de fluor et/ou de chlore, enfin le chlore ou le brome, et

R'' peut prendre les valeurs mentionnées pour R', mais à condition que la somme des atomes de carbone des restes alkyle, alcényle et alkoxy, pour R' et R'' ensemble, reste inférieure d'une unité au nombre d'atomes de carbone qui est indiqué ci-dessus dans la définition de R pour les restes mentionnés, et qu'au maximum l'un des restes R' et R'' représente un groupe phényle éventuellement substitué comme indiqué ou un groupe halogénométhyle,

en présence d'un solvant et le cas échéant en présence d'un catalyseur, ou

d) avec des isocyanates de formule générale VI

$$O=C=N-R''' \qquad (VI)$$

dans laquelle

R''' est un groupe alkyle ayant jusqu'à 12 atomes de carbone, un groupe halogénalkyle ayant jusqu'à 4 atomes de carbone et jusqu'à 5 atomes d'halogènes, un groupe alkoxycarbonyle ou un groupe alkoxyalkyle ayant chacun jusqu'à 4 atomes de carbone dans chaque partie alkyle, ou un groupe phényle qui peut être substitué par un halogène, un radical alkyle ayant jusqu'à 4 atomes de carbone, un radical alkoxy et un radical alkylthio ayant jusqu'à 2 atomes de carbone et un radical halogénalkyl ayant jusqu'à 2 atomes de carbone et jusqu'à 5 atomes d'halogènes, en présence d'un solvant et, le cas échéant, en présence d'un catalyseur, et

on transforme encore, le cas échéant, les composés obtenus selon les procédés a à d par réaction avec des acides en les sels ou par réaction avec des sels métalliques, en les complexes de sels métalliques correspondants.

3. Compositions fongicides, caractérisées par une teneur en au moins un dérivé de triazolyl-γ-fluoropinacolyle acylé de formule générale I suivant les revendications 1 et 2.

4. Utilisation de dérivés de triazolyl-γ-fluoropinacolyle acylés de formule générale I suivant les revendications 1 et 2 pour combattre des champignons dans la protection des plantes, dans la mesure où il ne s'agit pas de médecine humaine et de médecine vétérinaire.

5. Procédé de production de compositions fongicides, caractérisé en ce qu'on mélange des dérivés de triazolyl-γ-fluoropinacolyle acylés de formule générale I suivant les revendications 1 et 2 avec des diluants et/ou des agents tensio-actifs.